# EUROPEAN PATENT SPECIFICATION

(11) **EP 4 201 372 B1**
(45) Date of publication and mention of the grant of the patent: **09.10.2024**
(21) Application number: 22213691.3
(22) Date of filing: 15.12.2022
(51) Int. Cl.: A61C 9/00, G01B 11/25

(54) **SCANNER COMPRISING A NOVEL LENS DRIVE MECHANISM**
SCANNER MIT NEUARTIGEM LINSENANTRIEBSMECHANISMUS
SCANNER COMPRENANT UN NOUVEAU MÉCANISME D'ENTRAÎNEMENT DE LENTILLE

(30) Priority: 22.12.2021 EP 21216829
(43) Date of publication of application: 28.06.2023
(60) Divisional application: 24197332.0
(73) Proprietor: 3Shape A/S, 1060 Copenhagen K (DK)
(72) Inventor: ANDERSEN, Mads Grønlund, 1060 Copenhagen K (DK); HANSEN, Finn, 1060 Copenhagen K (DK)
(74) Representative: Guardian IP Consulting I/S

(56) References cited:
- WO-A1-2018/168635
- US-A- 4 935 635
- US-A- 5 424 836

## Description

### TECHNICAL FIELD

The present disclosure relates, generally, to an intra-oral scanner. More particularly, the disclosure relates to an intra-oral scanner having a mechanical system configured to linearly move at least one lens to change a focal plane of a lens system of the scanner.

### BACKGROUND

Intraoral scanners are generally used to capture direct digital impression of a patient's teeth directly from patient's oral cavity. The scanner projects light onto dental area to be scanned. 2 dimensional images captured by imaging sensors in response to the projected light are processed such as by a scanning software to generate a 3D digital representation of the scanned dental area.

In both prosthodontic and orthodontic procedures, obtaining surficial 3D digital representation of a dental object in the oral cavity may be a first step in performing a diagnosis or treatment. The more complete and accurate the scans of the dental object are, the higher the quality of the surficial 3D digital representation, and thus the greater the ability to design an optimal prosthesis or orthodontic treatment appliance. However, in the existing intraoral scanners, it is difficult to change a focal plane to capture images of the dental object at different focal planes, which limits the quality and completeness of 3D digital representation of the dental object. US 4 935 635 A discloses an intra-oral handheld 3D optical scanner.

It is desired to have a scanner comprising a mechanical system such as a drive mechanism configured to linearly move at least one lens to change the focal plane of a lens system to capture images of dental object.

### SUMMARY

An intraoral scanner may include an optical probe (i.e. scanner tip) configured to be moved within an oral cavity of a patient during scanning of dental object(s) within the oral cavity, an illumination module comprising at least one light source configured to generate illumination signal (e.g. incident light) for illuminating the dental object within the oral cavity, and focusing optics configured to focus the illumination signal to a focal plane external to the optical probe. The focal plane may be orthogonal or non-orthogonal to a direction of propagation of the incident light. The scanner may further include an image sensor configured to obtain data in response to the illumination of the dental object. The obtained data includes a 2D image that correspond to characteristic(s) of a dental object being scanned. The characteristics includes at least one of surface topology or texture data such as color data of the dental object. A processor, either comprised in the scanner or at least partly remote from the scanner, is configured to determine the surface topography and/ or texture data of the patient's teeth based on the obtained data. The focusing optics typically includes a lens assembly and for a specific position of the lens assembly, the focal plane may be fixed relative to the optical probe during scanning of the dental object.

One aspect of the disclosure is to provide an intraoral scanner that is configured to change a focal plane to allow obtaining two dimensional images of a dental object at various focal planes.

One aspect of the disclosure is to provide an intraoral scanner having a mechanical system that is configured to move a lens assembly to change the focal plane of the intraoral scanner. For a specific position of the lens assembly comprising a lens during scanning of the dental object, the focal plane may be fixed relative to the optical probe.

One aspect of the disclosure is to provide a drive assembly having a drive (e.g., electric motor) and a guide that is configured to be rotated by the drive. The rotation of the drive allows for back-and-forth movement of the lens assembly about a translation axis, preferably without changing a direction of rotation of the drive. The motion of the lens assembly positions the lens assembly at different positions along the translation axis, thus allowing changing the position of the focal plane and obtaining two dimensional images corresponding to different positions of the focal plane.

One aspect of the disclosure is to provide an intraoral scanner having reduced friction during back-and-forth movement of the lens assembly. This may be achieved, for example by providing lubrication between components that interface during movement such as between one or more rails and the lens housing or between coupling element and guide. This provides a smooth motion of the lens assembly, thereby allowing to change the focal plane.

One aspect of the disclosure is to provide a coupling element for engaging the guide with the lens housing. The coupling element, especially when operationally coupled with a biasing member such as a spring, reduces vibration during transfer of motion from the drive assembly to the lens assembly.

At least some of the above recited aspects are provided by an intra-oral handheld 3D optical scanner. The scanner includes an illumination module configured to generate an illumination signal to illuminate a dental object, and an image sensor configured to obtain data in response to the illumination of the dental object. The data is configured to be used to generate a 3D dental model of the dental object. The scanner also includes a lens housing comprising an optical lens that is configured to direct the illuminating signal towards the dental object. The scanner further includes a drive comprising a shaft that is configured to rotate around a rotation axis, a guide extending continuously along at least a part of a length of the shaft, and a spring arranged intermediate between the lens housing and the guide. The spring is configured to exert a spring force towards the guide.

The optical lens may further be configured to direct the reflected illuminating signal from the illuminated dental object towards an image sensor.

In some aspect, the drive assembly is supported/mounted on the frame/ housing130 of the scanner by using suitable brackets.

In some embodiments, the illumination signal is a light or a structured light. The light may include a white light or a plurality of colored light sources. The plurality of colored light sources (e.g. RGB) may typically be switched on sequentially during scanning of the dental object.

In some embodiments, the structured light includes a pattern corresponding to at least one of a physical structure introduced in light path between at least a light source of the illumination module and dental object, a digitally generated light pattern, or relative arrangement of more than one light source of the illumination module. The pattern may include different patterns such as periodic pattern of parallel lines or dots extending in a plane perpendicular to the optical axis such as checkerboard pattern with alternating relatively brighter and relatively darker regions.

The structured light facilitates in determining/obtaining surface geometry information and surface color information of the dental object. Use of the structured light in context of intraoral optical scanner is disclosed in US application number 16/774843 assigned to 3Shape AS.

In some embodiments, a length of the shaft is along the rotation axis.

In some embodiments, a longitudinal axis of the shaft is parallel to an optical axis of the optical lens.

In some embodiments, the guide extends continuously around the circumferential surface of the shaft.

In some embodiments, the guide is defined by a closed path.

In some embodiments, the guide is defined by the closed path that is non-parallel to the rotation axis of the shaft. The closed path is generally understood as a path having a start point and end point that are same. The start point and end point of the guide correspond to one back and forth linear movement of the lens assembly along the translation axis, i.e. the lens assembly moves from one extreme point to another extreme point and back from the another extreme point to one extreme point along the translation axis.

In some embodiments, the guide is defined by the closed path such that a 2D projection of the closed path is non-parallel to the rotation axis. The 2D projection is in a direction perpendicular to the rotation axis.

The closed path of the guide that is non-parallel to the rotation axis of the shaft enables back and forth (i.e., reciprocating motion of the lens housing) between two extreme positions without changing a direction of rotation of the drive. Accordingly, reducing the vibrations of the lens assembly during scanning of the dental object.

In some embodiments, the guide extends sinusoidally around the circumferential surface.

In some embodiments, the shaft comprises an end part connected coaxially with rest of the shaft and the guide is arranged on the end part. The end part may include a part that is detachably connected to the rest of the shaft. Alternatively, the end part may be an integral portion of the shaft, and the guide is defined along an outer circumferential surface of the shaft. The end part is adapted to rotate along the shaft around the rotational axis. The end part and the rest of the shaft are typically made up of different materials.

In some embodiments, the guide extends continuously to form a closed sinusoidal curve around the circumferential surface of the shaft.

In some embodiments, the shaft is configured to rotate around the rotation axis for producing a linear movement of the lens housing along a translation axis.

In some embodiment, linear movement of the lens housing is based on cylindrical cam-follower mechanism. The shaft comprising the guide may represent the cylindrical cam and the spring loaded coupling may represent the follower.

In some embodiments, the linear movement comprises back and forth movement between a first extreme position and a second extreme position.

In some embodiments, a distance of the linear movement of the lens housing corresponds to the length of the guide around the circumferential surface of the shaft.

In some embodiments, the spring is arranged such that the longitudinal axis of the spring is orthogonal to the optical axis of the optical lens.

In some embodiments, the spring is arranged such that the longitudinal axis of the spring is spatially separated relative to the optical axis of the optical lens.

In some embodiments, the spring is arranged such that the longitudinal axis of the spring is normal to the rotation axis.

In some embodiments, the lens housing comprises an attachment part where the spring fixedly attaches with the lens housing.

In some embodiments, the attachment part comprises a gap comprising at least a part of the spring arranged therein.

In some embodiments, the attachment part comprises a plurality of protrusions, the plurality of protrusions at least partly encircles a free space. The spring is arranged in the free space and the spring being at least partly encircled by the protrusion.

In some embodiments, the lens housing comprises at least one pin extending from the lens housing, the spring being arranged such that the at least one pin extends along the longitudinal axis of the spring and supports the spring.

In some embodiments, the spring is configured to transfer a spring force towards the guide and the lens housing.

In some embodiments, the scanner includes a coupling element operationally connecting the guide with the spring.

In some embodiments, the spring applies a force configured to maintain a connection between the coupling element and the lens housing.

In some embodiments, the shaft is configured to rotate around the rotation axis. The coupling element is configured to move along the guide in response to the rotation of the shaft, and the lens housing is configured to linearly move between a first extreme position and a second extreme position along a translation axis in response to the movement of the coupling element along the guide.

In some embodiments, the spring applied force is configured to maintain a connection between the coupling element and guide.

In some embodiments, the spring applies a force configured to maintain a connection between the coupling element and lens housing during the movement of lens housing along the translation axis.

In some embodiments, the spring applies force configured to maintain a connection between the coupling element and the guide during the movement of lens housing along the translation axis.

In some embodiments, the spring is configured to transfer a spring force between the coupling element and the lens housing.

In some embodiments, the spring is configured to transfer a lateral force between the coupling element and the lens housing.

In some embodiments, the spring is configured to transfer an axial force component to maintain a physical contact between the coupling element and the guide in response to the rotation of the drive.

In some embodiments, the spring comprises a stiffness that allows transferring an axial force component to maintain a physical contact between the coupling element and the guide during the rotation of the drive to the lens housing.

In some embodiments, at least a part of the coupling element is arranged in the attachment part.

In some embodiments, at least a part of the spring and at least a part of the coupling element is at least partly encircled by the plurality of protrusions.

In some embodiments, the guide is one of a male part or a female part and the coupling element is another of the female part or male part.

In some embodiments, a female guide part is a groove.

In some embodiments, the groove comprises a V-groove.

In some embodiments, the male coupling element part is a ball element configured to operationally interact with the female guide part.

In some embodiments, the male guide part is a protruded structure.

In some embodiments, the female coupling element part comprises a slot configured to operationally interact with the male guide part.

In some embodiments, lubrication is provided at an interface between the coupling element and the guide.

In some embodiments, the lens housing is configured to move along one or more rails.

In some embodiments, the lens housing includes a structure to house and support the focus lens that is configured to create focal planes.

In some embodiments, the lens housing includes at least one bracket attached to the ring structure and slidably supported on one or more rails. The at least one bracket is configured to slide along the one or more rails, thus allowing linear movement of the lens along the one or more rails to allow creating focus planes.

In some embodiments, the spring is arranged such that the longitudinal axis of the spring is normal to one of the one or more rails.

In some embodiments, the spring comprises a stiffness such that the spring allows for a backlash free connection between the coupling element and the guide.

In some embodiments, one or more rails is ferromagnetic.

In some embodiments, one or more magnets are arranged adjacent to the one or more rails.

In some embodiments, one or more magnets are arranged in or on the lens housing.

In some embodiments, the one or more magnets are configured to mitigate vibration of the lens housing during movement of the lens housing along the one or more rails.

In some embodiments, the magnetic flux density of the one or more magnets is at least 0.1 Tesla, such as between 0.1 - 5 Tesla.

In some embodiments, lubrication is provided between the one or more rails and the lens housing.

In some embodiments, the lens housing is configured to slide along at least two rails, and the lens housing interfaces at two contact surfaces with at least one rail of the at least two rails.

In some embodiments, the lens housing is configured to slide along at least two rails, and the lens housing interfaces at one contact surface with at least one rail of the at least two rails.

### BRIEF DESCRIPTION OF THE ACCOMPANYING DRAWINGS

Having thus described example embodiments of the present disclosure in general terms, reference will now be made to the accompanying drawings, which are not necessarily drawn to scale, and wherein:
FIG. 1 illustrates a schematic view of an intra-oral scanner, in accordance with an embodiment of the disclosure;
FIG. 2 illustrates a perspective view of a part of the intra-oral scanner in accordance with an embodiment of the disclosure;
FIG. 3 illustrates a perspective view of the lens assembly mounted on a pair of rails and arranged inside the housing of the intra-oral scanner, in accordance with an embodiment of the disclosure;
FIG. 4 illustrates a perspective view of the lens assembly operatively engaged with the drive assembly via a coupling element, in accordance with an embodiment of the disclosure;
FIG. 5 illustrates a side view of the lens assembly engaged with the drive assembly via the coupling element, in accordance with an embodiment of the disclosure;
FIG. 6 illustrates a sectional perspective view of the lens assembly engaged with the drive assembly and depicting the coupling arranged inside an attachment part of the housing and engaged with a guide, in accordance with an embodiment of the disclosure;
FIG. 7 illustrates an exploded view of the lens assembly and rails disengaged from a lens housing of the lens assembly, in accordance with an embodiment of the disclosure;
FIG. 8 illustrates a perspective view of the lens housing of the lens assembly, in accordance with an embodiment of the disclosure;
FIG. 9 illustrates a perspective view of the end part having the guide as a groove , in accordance with an embodiment of the disclosure;
FIG. 10 illustrates a sectional view of the end part depicting a V-shape of the groove, in accordance with an embodiment of the disclosure; and
FIG. 11 illustrates a front sectional view of the lens assembly supported on the rails and depicting the lens housing having two contact surfaces with a first rail and one contact surface with a second rail, in accordance with an embodiment of the disclosure.

### DETAILED DISCRIPTION

In the following description, for purposes of explanation, numerous specific details are set forth in order to provide a thorough understanding of the present disclosure. It will be apparent, however, to one skilled in the art that the present disclosure can be practiced without these specific details. In other instances, apparatus and methods are shown in block diagram form only in order to avoid obscuring the present disclosure.

Reference in this specification to "one embodiment" or "an embodiment" means that a particular feature, structure, or characteristic described in connection with the embodiment is included in at least one embodiment of the present disclosure. The appearance of the phrase "in one embodiment" in various places in the specification are not necessarily all referring to the same embodiment, nor are separate or alternative embodiments mutually exclusive of other embodiments. Some of these embodiments may appropriately be combined with one another. Further, the terms "a" and "an" herein do not denote a limitation of quantity, but rather denote the presence of at least one of the referenced items. Moreover, various features are described which may be exhibited by some embodiments and not by others. Similarly, various requirements are described which may be requirements for some embodiments but not for other embodiments.

Some embodiments of the present disclosure will now be described more fully hereinafter with reference to the accompanying drawings, in which some, but not all, embodiments of the disclosure are shown. Like reference numerals refer to like elements throughout.

The embodiments are described herein for illustrative purposes and are subject to many variations. It is understood that various omissions and substitutions of equivalents are contemplated as circumstances may suggest or render expedient but are intended to cover the application or implementation without departing from the scope of the present invention as defined by the appended claims. Further, it is to be understood that the phraseology and terminology employed herein are for the purpose of the description and should not be regarded as limiting. Any heading utilized within this description is for convenience only and has no legal or limiting effect.

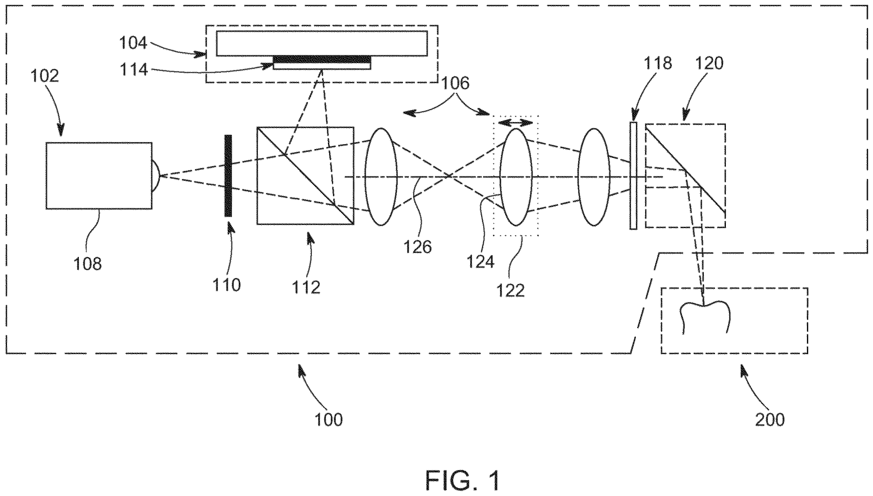

Referring to FIG. 1, a schematic view of an intra-oral handheld 3d scanner 100 (hereinafter referred to as scanner 100) according to an embodiment of the disclosure is shown. As shown, the scanner 100 includes an illumination module 102 configured to generate an illumination signal to illuminate a dental object 200 within an oral cavity, i.e. mouth, of a patient, an image sensor 104 to capture images of the dental object 200, and a lens system 106 to focus/direct the illumination signal from the illumination module 102 towards the dental object 200 and images the dental object 200 on the image sensor 104. In an embodiment, the image sensor 104 obtains data in response to the illumination of the dental object 200 via the lens system 106 and the data is used to generate a 3D dental model of the dental object 200. In some embodiments, the data is in the form of 2D images of the dental object 200.

The illumination signal may be a light or a structured light. In some embodiments, the structured light may include a pattern corresponding to at least one of a physical structure introduced in light path between at least a light source 108 of the illumination module 102 and the dental object 200, a digitally generated light pattern, or relative arrangement of more than one light source of the illumination module 102. To provide a structured light, in an embodiment, the illumination module 102 may include a pattern generating element 110 to incorporate a spatial pattern of light into a light beam generated by the at least one light source 108. As shown, the pattern generating element 110 may be arranged between the light source 108 and the lens system 106 to introduce a spatial pattern inside the light beam. As shown, the structured light may be provided by introducing/placing a suitable physical structure 110 in the path of the illumination signal provided by the at least one light source 108. In some embodiments, the structured light may be generated by arranging multiple light sources 108 in a suitable spatial arrangement.

In some embodiments, the scanner 100 may include a beam splitter 112 arranged between the pattern generating element 110 and the lens system 106. The beam splitter 112 facilitates in directing the reflected light received from the dental object 200 through the lens system 106 to the image sensor 104.

In an embodiment, the image sensor 104 includes a color filter array 114. Although drawn as a separate entity, the color filter 114 array is typically integrated with the image sensor 104, with a single-color filter for every pixel. Moreover, the scanner may include polarization optics 118. Having the color filter array (e.g. Bayer filter) allows for simultaneously capturing data that may be used for both determining topology and color of the dental object, thereby allowing generation of 3D digital representation with color information of the dental object. Alternatively, instead of using a color filter array, the scanner may include multi-colored light sources (e.g. RGB) that are switched sequentially so that data corresponding to different colored light source may be captured. Any of the color data obtained by illuminating the dental object, using one of the multi-colored light sources, may be used to determine surface topology. Color data may be obtained by illuminating the dental object using each of the multi-colored light sources may be used to use specific color data. The specific color data and surface topology may be mapped to generate 3D digital representation with color information of the dental object.

Polarization optics 118 may be used to selectively image specular reflections and block out undesired diffuse signal from sub-surface scattering inside the scanned object 200. The scanner 100 may include folding optics, e.g. a mirror 120, which directs the light out in a direction different to the optical path of the lens system 106, e.g., in a direction perpendicular to the optical path of the lens system 106.

In an embodiment, the lens system 106 includes a lens assembly 122 having at least one optical lens 124. The lens system is adapted to move along an optical axis 126 of the lens system 106 to shift a focal plane of the lens system 106. The optical lens 124 (i.e., lens 124) is configured to direct the illumination signal towards the dental object 200. In an embodiment, the lens 124 may be a single lens, a doublet lens, or even a triplet lens.

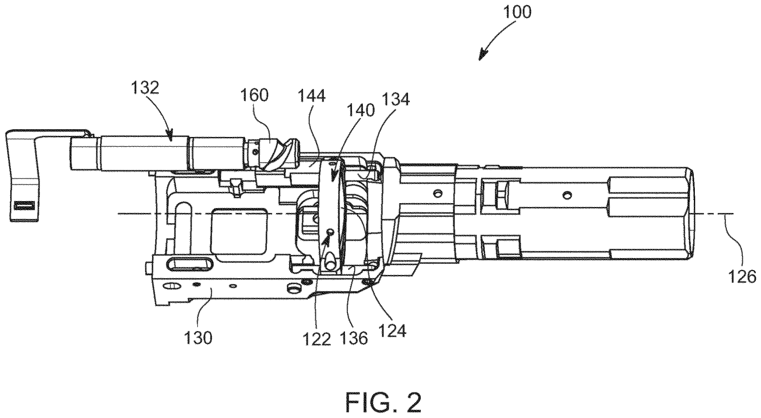

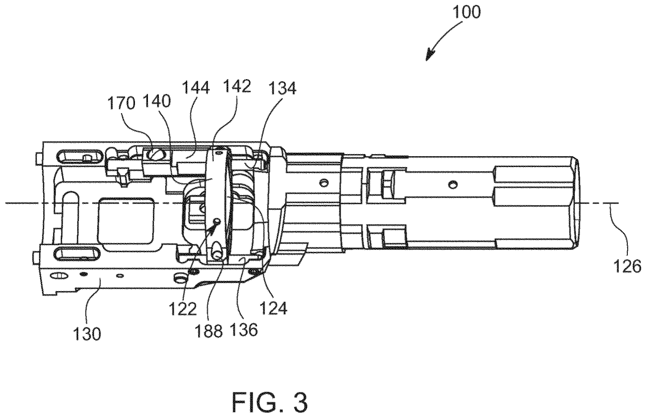

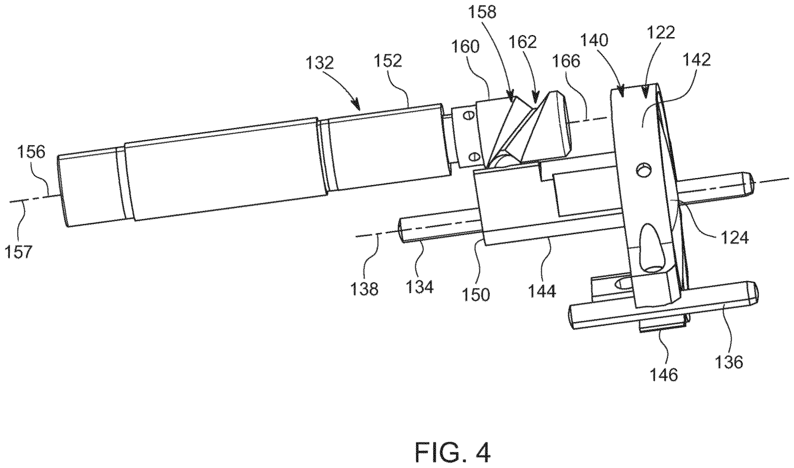

Referring now to FIGS. 2, which illustrates a part of the intraoral scanner 100 with some of the components of the intra-oral scanner removed and depicting a lens assembly 122 and a drive assembly 132 operatively engaged with the lens assembly arranged inside a housing of the intra-oral scanner according to an embodiment. The lens assembly 122 is arranged inside a frame 130 of the scanner 100 and operatively coupled to a drive assembly 132 of the scanner 100 is shown. The drive assembly 132 is supported/mounted on the frame 130 by using suitable brackets. As shown in FIGS. 2 to 6, the lens assembly 122 is mounted on at least one rail such as a pair of rails 134, 136 arranged inside the frame 130 and is adapted to move linearly back and forth between a first extreme position and a second extreme position along at least a part of respective lengths of the rails 134, 136. It may be appreciated that a translation axis (138, Fig. 4) of the lens assembly 122 is substantially parallel to the optical axis 126. The rails, for example, a first rail 134 and a second rail 136, are arranged spaced apart from each other and are supported by a supporting mechanism within the frame 130.

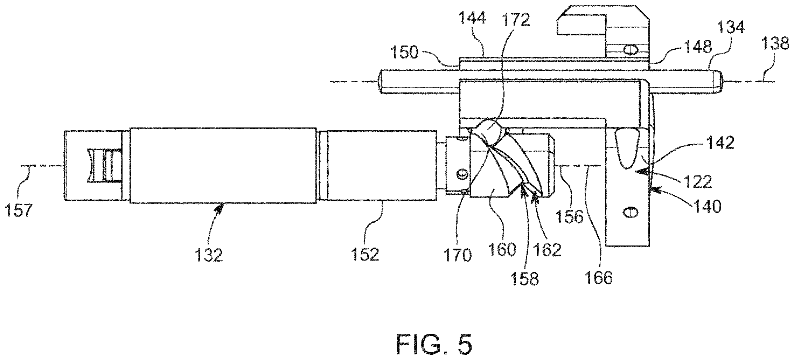

As best shown in FIGS. 4 to 8, the lens assembly 122 includes a lens housing 140 adapted to house and support the lens 124 and is movably arranged on the rails 134, 136. In an embodiment, the lens 124 is attached, e.g. adhesively coupled, to the lens housing 140 and is arranged such that the lens 124 is aligned to optical axis (126, Fig. 1-3). The lens assembly 122 may include a magnetic encoder to determine a position of the lens assembly 122 relative to rails 134, 136, the determined position may be associated with a focal plane and used to generate 3D digital representation of dental object from the oral cavity. As shown, the lens housing 140 may include a ring structure 142 to house and support the lens 124, and a pair of brackets, for example, a first bracket 144 and a second bracket 146, attached to the ring structure 142 and slidably supported on the rails 134, 136. The first bracket 144 and the second bracket 146 are supported on the first rail 134 and the second rail 136 respectively, and extend from the ring structure 142 in a direction, such as radial direction. The brackets 144, 146 may also be substantially parallel to a central longitudinal axis of the ring structure 142. Moreover, a length of the two brackets may be equal or different with one bracket longer than the other, such as the first bracket 144 being greater than a length of the second bracket 146. Each of the brackets 144, 146 may include substantially semicircular cross-section when looking from a front of the lens housing 140. The first bracket 144 includes a first longitudinal end (148, Fig. 5) attached to the ring structure 142 and a second longitudinal end (150, Fig. 5) arranged away from the ring structure 142 and defines a free end of the first bracket 144. The second longitudinal end 150 is arranged proximate to the drive assembly 132 and may be operatively coupled to the drive assembly 132. In an embodiment, the first bracket 144, the second bracket 146, and the ring structure 142, may be constructed as one part.

Referring to FIGS. 4 to 7, the drive assembly 132 includes a drive 152, for example, an electric motor, having a drive shaft 154 (i.e., shaft 154) operatively engaged with the first bracket 144 (i.e., the lens housing 140). The first bracket 144 is engaged with the drive 152 such that a rotation of the shaft 154 around its rotation axis 156 causes a translation movement of the lens housing 140 along the translation axis 138 disposed parallel to the optical axis 126 of the lens system 106. In the illustrated embodiment, the rotation of the shaft 154 about the rotation axis 156 causes a back-and-forth movement of the lens housing 140 (i.e., the lens assembly 122) between the first extreme position and the second extreme position.

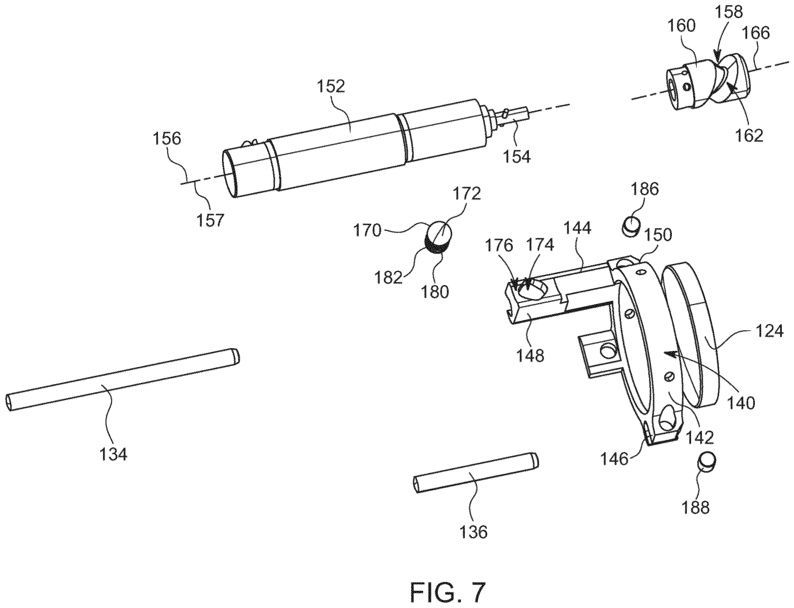

As shown in FIG. 4 to 7, a length of the shaft (154, Fig. 7) is along the rotation axis 156 and coincides with a central longitudinal axis 157 of the shaft 154. Further, the longitudinal axis (157, Fig. 7) of the shaft 154 is parallel to the optical axis 126. To operatively engage the lens housing 140 (i.e., the first bracket 144) with the shaft 154, the drive assembly 132 includes a guide 158 arranged or defined continuously along an outer circumferential surface of an end part 160 of the shaft 154. In the illustrated embodiment, the end part 160 is a separate component and is removably attached, as attached for example by a pin-locking mechanism, at an end of the shaft 154 and is arranged coaxially to the shaft 154. However, it may be appreciated that the end part 160 may be an integral portion of the shaft 154, and the guide 158 is defined along an outer circumferential surface of the shaft 154. The end part 160 is adapted to rotate along the shaft 154 around the rotational axis 156.

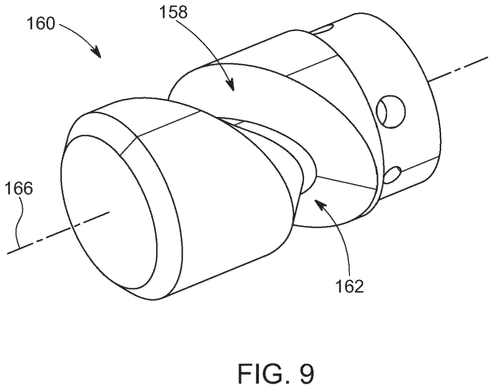

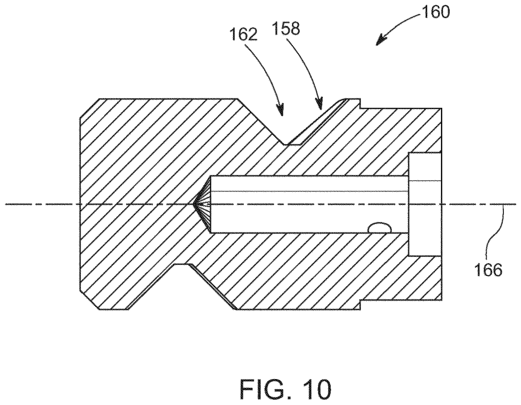

As shown, the guide 158 is a groove 162 extending continuously along the outer circumferential surface of the end part 160. As shown, the guide 158 is a female guide and defines a closed path on the outer circumferential surface of the end part 160. As shown in FIGS. 4 to 7, 9, and 10, the groove 162 is defined along the outer circumference surface such that the closed path of the groove 162 is arranged at an angle that is greater than 0 degrees and less than 90 degrees relative to the rotation axis 156. Accordingly, the closed path of the groove 162, and hence the guide 158, is non-parallel to a central longitudinal axis 166 of the end part 160, and hence is non-parallel to the rotation axis 156. Accordingly, the guide 158 is defined by the closed path such that a 2D projection of the closed path is non-parallel to the rotation axis 156. In some embodiments, the groove 162 extends in an elliptical manner along the outer circumferential surface of the end part 160 such that a plane of the ellipse having both major axis and minor axis are at an angle relative to the rotation axis 156 and the central axis 166 of the end part 160. Also, in some embodiments, the guide 158, and hence the groove 162, follows a path of a sinusoidal curve around the outer circumferential surface of the end part 160. Accordingly, the guide 158, and hence the groove 162, extends continuously to form a closed sinusoidal curve around the circumferential surface of the shaft 154 or the end part 160 of the shaft 154. In an embodiment, the groove 162 is V-shaped groove (as best shown in FIG. 10).

It may be appreciated that distance of the linear movement of the lens housing 140 (i.e., lens assembly 122) between the first extreme position and second extreme position corresponds to a length of the closed path of the guide 158 around the circumferential surface of the shaft (e.g. end part 160) along which the coupling member travels from a start point to end point of the closed path. The distance of the linear movement of the lens housing 140 is lesser than half the length of the closed path guide 158 around the circumferential surface of the shaft (e.g. end part 160). The distance of the linear movement of the lens housing 140 in one direction during the back-and-forth movement equals to an axial distance between the two most distant points on the guide that is located at the circumferential surface of the shaft. The axial distance may be defined along the rotational axis of the shaft.

Although the guide 158 is described as the closed path, it may be envisioned that guide may include an open path. In such a case, the lens housing 140 (i.e., the lens assembly 122) is moved back and forth by rotating the shaft 154, and hence the end part 160, in both directions. For open path guide, this may be performed by configuring the motor to change rotational direction of the shaft to allow back and forth movement of the lens housing. Alternatively, for open path guide, this may be performed by having two motors, one motor configured to rotate the shaft in a first rotational direction and another motor configured to rotate the shaft in a second rotational direction opposite to the first rotational direction to allow back and forth movement of the lens housing.

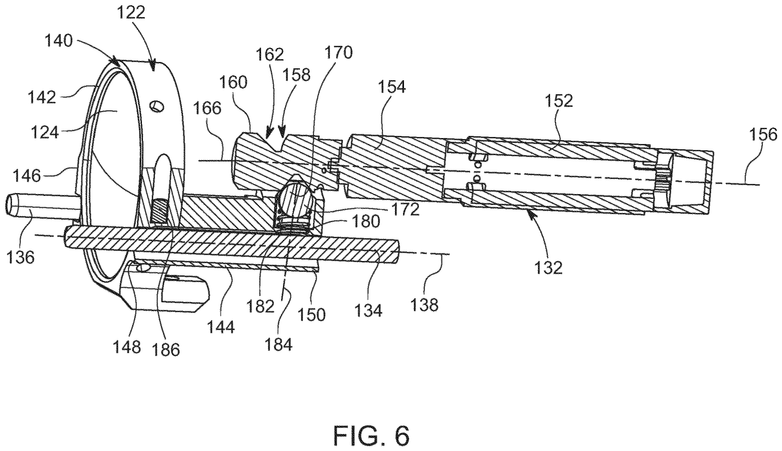

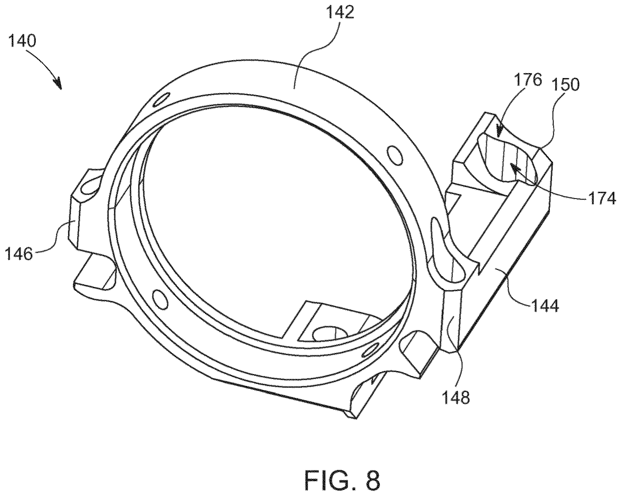

Additionally, as shown in FIGS. 4 to 7, the scanner 100 includes a coupling element 170 as a male part for operatively engaging the end part 160 with the first bracket 144 (i.e., lens housing 140). The coupling element 170 is adapted/arranged to move along the guide 158 in response to the rotation of the guide 158, and hence the end part 160, to enable the translation movement of the lens housing 140 along the translation axis 138. As shown, the coupling element 170 is a spherical ball 172 arranged partially inside the guide 158 and partially inside a gap 174 defined by an attachment part 176 extending from the first bracket 144, as best shown in FIG. 6. As best shown in FIGS. 6 -8, the attachment part 176 is arranged proximate to the second longitudinal end 150 of the first bracket 144 and extends radially outwardly/inwardly from an outer surface of the first bracket 144. As best shown in FIG. 8, the attachment part 176 is in the form a hollow portion extending inwardly from an outer surface of the first bracket 144 and defining a free space (i.e., the gap 174) therebetween to receive a part of the coupling element 170, thereby supporting the coupling element 170. In some embodiments, the attachment part 176 may include a plurality of protrusions arrayed circularly and encircling a free space to define the gap 174 therebetween for receiving a part of the coupling element 170. In such a case, the protrusions are arranged spaced apart from each other and extend outwardly from the outer surface of the first bracket 144.

Also, the coupling element 170 exerts a force on the attachment part 176 as the coupling element 170 travels along the guide 158 to enable the translational movement of the lens assembly 122 (i.e., the lens housing 140) along the translation axis 138 between the first extreme position and the second extreme position. Although the coupling element 170 is contemplated as the spherical ball 172, it may be envisioned that that the coupling element 170 may be a cylindrical pin, or having any other geometrical shape that is designed to slidably engage with the guide. It may be appreciated that a height of the attachment part 176 and a width of the gap 174 are selected such that a portion of the coupling element 170 is arranged inside the gap 174. Although the guide 158 is shown and contemplated as a female part and the coupling element 170 is shown and contemplated as a male part, it may be appreciated that the guide 158 may be a male part, for example a protruded structure, and a coupling element 170 may be a female part, for example, a slot in which the protruded structure extends. To enable smooth movement of the coupling element 170 along the path of the guide 159, lubrication is provided at an interface between the coupling element 170 and the guide 158.

To keep the coupling element 170 engaged with the guide 158 and to facilitate a smooth movement of the coupling element 170 along the path of the guide 158, the lens assembly 122 includes a biasing member 180, for example, a spring 182 (best shown in FIGS. 6 and 7). Referring to FIG. 6, the spring 182 is configured to apply spring force to keep a part of the coupling element 170 engaged with the guide 158 (i.e., inside the groove 162). Accordingly, the spring 182 facilitates in maintaining the engagement of the coupling element 170 with the guide 158 as coupling element 170 moves along the path defined by the guide 158. As shown, the spring 182 is arranged inside the gap 174 defined by the attachment part 176 such that spring 182 is arranged between the outer surface of the first bracket 144 (i.e., the lens housing 140) and the coupling element 170, and exerts a biasing force towards the end part 160 (i.e., guide 158) of the shaft 154 to retain the coupling element 170 engaged with the guide 158. In an embodiment, an end of the spring 182 is fixedly attached with the first bracket 144, and hence the lens housing 140, and other end of the spring 182 is engaged with the coupling element 170.

As shown, the coupling element 170 rests on the spring 182 and compresses the spring 182. Accordingly, spring 182 exerts a force towards the guide 158 and the lens housing 140 (i.e., first bracket 144) in an axial direction. Also, the spring 182 is configured to transfer a lateral force between the coupling element 170 and the lens housing 140 to enable the linear movement of lens housing 140 between the first extreme position and the second extreme position. Further, the spring 182 and the attachment part 176 are disposed such that a longitudinal axis 184 of the spring 182 and the optical axis 126 are arranged in different plane, and therefore, the longitudinal axis 184 and optical axis 126 are arranged separated from each other. Also, the spring 182 is arranged such that the longitudinal axis 184 of the spring is arranged orthogonal (i.e., normal) to the optical axis 126. Also, the longitudinal axis 184 is substantially perpendicular to the rotation axis 156. Also, the longitudinal axis 184 is substantially normal to the first rail 134. It may be appreciated that a stiffness of the spring 182 is selected such that the connection between the coupling element 170 (i.e., ball 172) and the guide 158 (i.e., groove 162) is a backlash free connection.

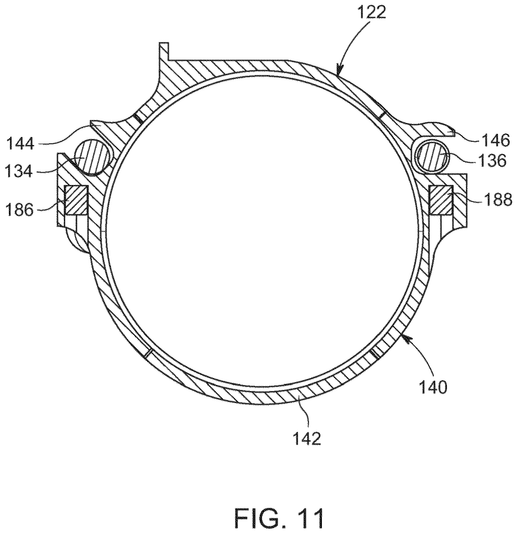

Additionally, to mitigate vibrations, magnets are provided in the lens housing 140 in the immediate vicinity of the first and second rails 134, 136, which in an embodiment, are made of ferromagnetic material. For example, as best shown in FIG. 11, a first magnet 186 is removably or permanently arranged inside the lens housing 140 and is disposed proximate to the first end 148 of the first bracket 144 and the first rail 134, while a second magnet 188 is removably or permanently arranged inside the lens housing 140 and extends inside the second bracket 146 and proximate to the second rail 136. Further, both the rails 134, 136 are made of ferromagnetic material to enable a magnetic coupling of the magnets 186, 188 with respective rails 134, 136. In some embodiments, to mitigate vibrations and to magnetically couple and support the lens housing 140 on the rails 134, 136, the magnetic flux density of the one or more of the magnets 186, 188 is between 0.1 Tesla and 5 Tesla depending on the size and the weight of the lens assembly 122.

In an embodiment, the first and second brackets 144, 146 are arranged relative to the rails 134, 136 so as to reduce contact area between the first and second brackets 144, 146 and the associated rails 134, 136. In an embodiment, the first and second brackets 144, 146 and the rails 134, 136 are arranged relative to each other such that line contacts or point contacts exist between the first and second brackets 144, 146 and the rails 134, 136. As shown in FIG. 11, the lens housing 140 (i.e., the first bracket 144) interfaces at two contact surfaces with the first rail 134, while the lens housing 140 (i.e., the second bracket 146) interfaces at one contact surface with the second rail 136). Further, to reduce the friction between the lens housing 140 and the rails 134, 136, suitable lubrication is provided between the rails 134, 136 and the brackets 144, 146.

A working of the scanner 100 to change a focal plane of the lens system 106 is now described. For moving focal plane of the lens 124 along the optical axis 126, the lens assembly 122 is moved linearly and back and forth between the first extreme position and the second extreme position along the translation axis. In the first extreme position, the lens 124 is arranged proximate to the illumination module 102 relative to the second extreme position. For moving the lens assembly 122 (i.e., lens housing 140 along with the lens 124), the drive 152 is powered, causing the rotation of the drive shaft 154 about its rotation axis. 156 In response to the rotation of the drive shaft 154, the end part 160 also rotates about the rotation axis 156. As the ball 172 (i.e., coupling element 170) is arranged inside the groove 162 (i.e., the guide 158), the ball 172 moves along the closed path defined by the groove 162 in response to the rotation of the end part 160.

As the groove path is inclined relative to the rotation axis 156 and the path being a closed path, the ball 172 moves/translates in a first direction (i.e., forward direction) towards the drive 152 during one half of each rotation of the end part 160 about the rotation axis 156, and moves/translates in a second direction (i.e., a rearward direction) away from the drive 152 during remaining half of the rotation of the end part 160, or vice versa, where the first direction is away from the drive, and the second direction is towards the drive. Due to the back-and-forth motion of the ball 172 along the guide 162, the ball 172 exerts a force on the attachment part 176, and hence the lens housing 140, causing the back-and-forth movement of the lens assembly 122 relative to the rails 134, 136 along the translation axis 138. Accordingly, the lens assembly 122 moves towards the one extreme position when the ball 172 moves in the first direction, and the lens assembly 122 moves towards another extreme position opposite to the one extreme position when the ball 172 moves in the second direction. In this manner, by moving the lens assembly 122 back and forth along the translation axis 138, the focal plane of the lens system 106, and hence the scanner 100 can be changed. This allows for acquiring a plurality of two-dimensional images of the dental object at different focal planes. It may be appreciated that the scanner 100 may be orientated at various orientation and the focal plane of the scanner 100 is changed by moving the lens assembly 122 at each orientation to take a stack of 2D images for each orientation. These 2D images are stitched together by applying known techniques such as Iterative Closest Point (ICP) to obtain three-dimensional digital representation of the dental object 200.

Many modifications and other embodiments of the disclosures set forth herein will come to mind to one skilled in the art to which these disclosures pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

### LIST OF ELEMENTS

- 100: scanner
- 102: illumination module
- 104: image sensor
- 106: lens system
- 108: light source
- 110: pattern generating element
- 112: beam splitter
- 114: color filter array
- 118: polarization optics
- 120: mirror
- 122: lens assembly
- 124: lens
- 126: optical axis
- 130: frame
- 132: drive assembly
- 134: first rail
- 136: second rail
- 138: translation axis
- 140: lens housing
- 142: ring structure
- 144: first bracket
- 146: second bracket
- 148: first end
- 150: second end
- 152: drive
- 154: shaft
- 156: rotation axis
- 167: longitudinal axis
- 158: guide
- 160: end part
- 162: groove
- 166: longitudinal axis
- 170: coupling element
- 172: spherical ball
- 174: gap
- 176: attachment part
- 180: biasing member
- 182: spring
- 184: longitudinal axis
- 186: first magnet
- 188: second magnet
- 200: dental object

## Claims

1. An intra-oral handheld 3D optical scanner (100) comprising:
an illumination module (102) configured to generate an illumination signal to illuminate a dental object (200);
an image sensor (104) configured to obtain data in response to the illumination of the dental object, the data being configured to be used to generate a 3D dental model of the dental object;
a lens housing (140) comprising an optical lens (124) that is configured to direct the illuminating signal towards the dental object;
a drive (152) comprising a shaft (154) that is configured to rotate around a rotation axis (156);
a guide (158) extending continuously along at least a part of a length of the shaft; **characterised by**
a spring (182), arranged intermediate between the lens housing and the guide, configured to exert a spring force towards the guide.

2. The intra-oral handheld 3D optical scanner according to claim 1, wherein the shaft comprises an end part (162) connected coaxially with rest of the shaft and the guide is arranged on the end part.

3. The intra-oral handheld 3D optical scanner according to any of the previous claims, wherein the guide extends continuously to form a closed sinusoidal curve around the circumferential surface of the shaft.

4. The intra-oral handheld 3D optical scanner according to any of the previous claims, wherein the shaft is configured to rotate around the rotation axis for producing a linear movement of the lens housing along a translation axis (138), and wherein the linear movement comprises back and forth movement between a first extreme position and a second extreme position.

5. The intra-oral handheld 3D optical scanner according to any of the previous claims, wherein the lens housing comprises an attachment part (176) where the spring fixedly attaches with the lens housing.

6. The intra-oral handheld 3D optical scanner according to any of the previous claims, wherein the attachment part comprises a plurality of protrusions, the plurality of protrusions at least partly encircles a free space, wherein the spring is arranged in the free space and the spring being at least partly encircled by the plurality of protrusions.

7. The intra-oral handheld 3D optical scanner according to any of the previous claims, further comprising a coupling element (170) operationally connecting the guide with the spring

8. The intra-oral handheld 3D optical scanner according to claim 7, wherein the spring applies a force configured to maintain a connection between the coupling element and the lens housing.

9. The intra-oral handheld 3D optical scanner according to any of claims 7-8, wherein
the shaft is configured to rotate around the rotation axis;
the coupling element is configured to move along the guide in response to the rotation of the shaft; and
the lens housing is configured to linearly move between the first extreme position and second extreme position along a translation axis (138) in response to the movement of the coupling element along the guide.

10. The intra-oral handheld 3D optical scanner according to any of claims 7-9, wherein the spring applies a force configured to maintain a connection between the coupling element and guide.

11. The intra-oral handheld 3D optical scanner according to any of claims 7-10, wherein at least a part of the coupling element is arranged in the attachment part.

12. The intra-oral handheld 3D optical scanner according to any of claims 7-11, wherein the guide is one of a male part or a female part and the coupling element is another of the female part or male part.

13. The intra-oral handheld 3D optical scanner according to any of the previous claims, wherein the lens housing is configured to move along one or more rails.

14. The intra-oral handheld 3D optical scanner according to claim 13, wherein the one or more rails is ferromagnetic, and arranged adjacent to the one or more rails.

15. The intra-oral handheld 3D optical scanner according to any of claims 13 or 14, wherein
the lens housing is configured to slide along at least two rails; and
the lens housing interfaces at two contact surfaces with at least one rail of the at least two rails and the lens housing interfaces at one contact surface with at least one rail of the at least two rails.

## Patentansprüche

1. Intraoraler tragbarer optischer 3D-Scanner (100), umfassend:
ein Beleuchtungsmodul (102), das dazu konfiguriert ist, ein Beleuchtungssignal zum Beleuchten eines Dentalobjekts (200) zu erzeugen;
einen Bildsensor (104), der dazu konfiguriert ist, als Reaktion auf die Beleuchtung des Dentalobjekts Daten zu erhalten, wobei die Daten dazu konfiguriert sind, zum Erzeugen eines 3D-Dentalmodells des Dentalobjekts verwendet zu werden;
ein Linsengehäuse (140), das eine optische Linse (124) umfasst, die dazu konfiguriert ist, das Beleuchtungssignal auf das Dentalobjekt zu richten;
einen Antrieb (152), der eine Welle (154) umfasst, die dazu konfiguriert ist, sich um eine Rotationsachse (156) zu drehen;
eine Führung (158), die sich kontinuierlich entlang mindestens eines Teils einer Länge der Welle erstreckt, **gekennzeichnet durch**
eine Feder (182), die zwischen dem Linsengehäuse und der Führung angeordnet ist und dazu konfiguriert ist, eine Federkraft in Richtung der Führung auszuüben.

2. Intraoraler tragbarer optischer 3D-Scanner nach Anspruch 1, wobei die Welle ein Endteil (162) umfasst, das koaxial mit dem Rest der Welle verbunden ist, und die Führung an dem Endteil angeordnet ist.

3. Intraoraler tragbarer optischer 3D-Scanner nach einem der vorstehenden Ansprüche, wobei sich die Führung kontinuierlich erstreckt, um eine geschlossene Sinuskurve um die Umfangsfläche der Welle zu bilden.

4. Intraoraler tragbarer optischer 3D-Scanner nach einem der vorstehenden Ansprüche, wobei die Welle dazu konfiguriert ist, sich um die Rotationsachse zu drehen, um eine lineare Bewegung des Linsengehäuses entlang einer Translationsachse (138) zu erzeugen, und wobei die lineare Bewegung eine Hin- und Herbewegung zwischen einer ersten Extremposition und einer zweiten Extremposition umfasst.

5. Intraoraler tragbarer optischer 3D-Scanner nach einem der vorstehenden Ansprüche, wobei das Linsengehäuse ein Befestigungsteil (176) umfasst, wo die Feder fest an dem Linsengehäuse befestigt ist.

6. Intraoraler tragbarer optischer 3D-Scanner nach einem der vorstehenden Ansprüche, wobei das Befestigungsteil eine Vielzahl von Vorsprüngen umfasst, wobei die Vielzahl von Vorsprüngen mindestens teilweise einen freien Raum umgibt, wobei die Feder in dem freien Raum angeordnet ist und die Feder mindestens teilweise von der Vielzahl von Vorsprüngen umgeben ist.

7. Intraoraler tragbarer optischer 3D-Scanner nach einem der vorstehenden Ansprüche, der weiter ein Kopplungselement (170) umfasst, das die Führung funktional mit der Feder verbindet.

8. Intraoraler tragbarer optischer 3D-Scanner nach Anspruch 7, wobei die Feder eine Kraft ausübt, die dazu konfiguriert ist, eine Verbindung zwischen dem Kopplungselement und dem Linsengehäuse aufrechtzuerhalten.

9. Intraoraler tragbarer optischer 3D-Scanner nach einem der Ansprüche 7-8, wobei
die Welle dazu konfiguriert ist, sich um die Rotationsachse zu drehen;
das Koppplungselement dazu konfiguriert ist, sich als Reaktion auf die Drehung der Welle entlang der Führung zu bewegen; und
das Linsengehäuse dazu konfiguriert ist, sich als Reaktion auf die Bewegung des Kopplungselements entlang der Führung linear zwischen der ersten Extremposition und der zweiten Extremposition entlang einer Translationsachse (138) zu bewegen.

10. Intraoraler tragbarer optischer 3D-Scanner nach einem der Ansprüche 7-9, wobei die Feder eine Kraft ausübt, die dazu konfiguriert ist, eine Verbindung zwischen dem Kopplungselement und der Führung aufrechtzuerhalten.

11. Intraoraler tragbarer optischer 3D-Scanner nach einem der Ansprüche 7-10, wobei mindestens ein Teil des Kopplungselements in dem Befestigungsteil angeordnet ist.

12. Intraoraler tragbarer optischer 3D-Scanner nach einem der Ansprüche 7-11, wobei die Führung eines von einem männlichen Teil oder einem weiblichen Teil ist und das Kopplungselement ein anderes des weiblichen Teils oder männlichen Teils ist.

13. Intraoraler tragbarer optischer 3D-Scanner nach einem der vorstehenden Ansprüche, wobei das Linsengehäuse dazu konfiguriert ist, sich entlang einer oder mehrerer Schienen zu bewegen.

14. Intraoraler tragbarer optischer 3D-Scanner nach Anspruch 13, wobei die eine oder mehreren Schienen ferromagnetisch sind und neben der einen oder mehreren Schienen angeordnet sind.

15. Intraoraler tragbarer optischer 3D-Scanner nach einem der Ansprüche 13 oder 14, wobei
das Linsengehäuse dazu konfiguriert ist, entlang mindestens zweier Schienen zu gleiten; und
das Linsengehäuse an zwei Kontaktflächen an mindestens einer Schiene der mindestens zwei Schienen anschließt und das Linsengehäuse an einer Kontaktfläche an mindestens einer Schiene der mindestens zwei Schienen anschließt.

## Revendications

1. Scanner optique 3D portatif intra-oral (100) comprenant :
un module d'éclairage (102) configuré pour générer un signal d'éclairage pour éclairer un objet dentaire (200) ;
un capteur d'image (104) configuré pour obtenir des données à la suite de l'éclairage de l'objet dentaire, les données étant configurées pour être utilisées pour générer un modèle dentaire 3D de l'objet dentaire ;
un logement de lentille (140) comprenant une lentille optique (124) qui est configurée pour diriger le signal d'éclairage vers l'objet dentaire ;
un entraînement (152) comprenant un arbre (154) qui est configuré pour tourner autour d'un axe de rotation (156) ;
un guide (158) s'étendant de manière continue le long d'au moins une partie d'une longueur de l'arbre ; **caractérisé par**
un ressort (182), agencé entre le logement de lentille et le guide, configuré pour exercer une force de ressort vers le guide.

2. Scanner optique 3D portatif intra-oral selon la revendication 1, dans lequel l'arbre comprend une partie d'extrémité (162) reliée coaxialement au reste de l'arbre et le guide est agencé sur la partie d'extrémité.

3. Scanner optique 3D portatif intra-oral selon l'une quelconque des revendications précédentes, dans lequel le guide s'étend de manière continue pour former une courbe sinusoïdale fermée autour de la surface circonférentielle de l'arbre.

4. Scanner optique 3D portatif intra-oral selon l'une quelconque des revendications précédentes, dans lequel l'arbre est configuré pour tourner autour de l'axe de rotation pour produire un mouvement linéaire du logement de lentille le long d'un axe de translation (138) et dans lequel le mouvement linéaire comprend un mouvement de va-et-vient entre une première position extrême et une seconde position extrême.

5. Scanner optique 3D portatif intra-oral selon l'une quelconque des revendications précédentes, dans lequel le logement de lentille comprend une partie de fixation (176) dans laquelle le ressort est fixé à demeure au logement de lentille.

6. Scanner optique 3D portatif intra-oral selon l'une quelconque des revendications précédentes, dans lequel la partie de fixation comprend une pluralité de saillies, la pluralité de saillies entourent, au moins en partie, un espace libre, dans lequel le ressort est agencé dans l'espace libre et le ressort étant au moins partiellement entouré par la pluralité de saillies.

7. Scanner optique 3D portatif intra-oral selon l'une quelconque des revendications précédentes, comprenant en outre un élément de couplage (170) reliant de manière fonctionnelle le guide au ressort.

8. Scanner optique 3D portatif intra-oral selon la revendication 7, dans lequel le ressort applique une force configurée pour maintenir une liaison entre l'élément de couplage et le logement de lentille.

9. Scanner optique 3D portatif intra-oral selon l'une quelconque des revendications 7-8, dans lequel
l'arbre est configuré pour tourner autour de l'axe de rotation ;
l'élément de couplage est configuré pour se déplacer le long du guide à la suite de la rotation de l'arbre ; et
le logement de lentille est configuré pour se déplacer linéairement entre la première position extrême et la seconde position extrême le long d'un axe de translation (138) à la suite du mouvement de l'élément de couplage le long du guide.

10. Scanner optique 3D portatif intra-oral selon l'une quelconque des revendications 7-9, dans lequel le ressort applique une force configurée pour maintenir une liaison entre l'élément de couplage et le guide.

11. Scanner optique 3D portatif intra-oral selon l'une quelconque des revendications 7-10, dans lequel au moins une partie de l'élément de couplage est agencée dans la partie de fixation.

12. Scanner optique 3D portatif intra-oral selon l'une quelconque des revendications 7-11, dans lequel le guide est l'une d'une partie mâle ou d'une partie femelle et l'élément de couplage est l'autre de la partie femelle ou de la partie mâle.

13. Scanner optique 3D portatif intra-oral selon l'une quelconque des revendications précédentes, dans lequel le logement de lentille est configuré pour se déplacer le long d'un ou de plusieurs rails.

14. Scanner optique 3D portatif intra-oral selon la revendication 13, dans lequel les un ou plusieurs rails sont ferromagnétiques et agencés de manière adjacente aux un ou plusieurs rails.

15. Scanner optique 3D portatif intra-oral selon l'une quelconque des revendications 13 ou 14, dans lequel
le logement de lentille est configuré pour glisser le long d'au moins deux rails ; et
le logement de lentille assure l'interface au niveau de deux surfaces de contact avec au moins un rail des au moins deux rails et le logement de lentille assure l'interface au niveau d'une surface de contact avec au moins un rail des au moins deux rails.
